(19) Europäisches Patentamt

European Patent Office

Office européen des brevets

(11) Publication number: **0 291 334 B1**

## EUROPEAN PATENT SPECIFICATION

(45) Date of publication of patent specification: **08.09.93**  (51) Int. Cl.⁵: **A61K 7/32**, A61K 7/38

(21) Application number: **88304366.3**

(22) Date of filing: **13.05.88**

(54) **Transparent antiperspirant stick compositions.**

(30) Priority: **15.05.87 US 50607**

(43) Date of publication of application:
**17.11.88 Bulletin 88/46**

(45) Publication of the grant of the patent:
**08.09.93 Bulletin 93/36**

(84) Designated Contracting States:
**AT BE CH DE ES FR GB GR IT LI NL SE**

(56) References cited:
**GB-A- 2 064 363
US-A- 4 151 272
US-A- 4 518 582**

(73) Proprietor: **UNILEVER PLC
Unilever House Blackfriars P.O. Box 68
London EC4P 4BO(GB)**

(84) Designated Contracting States:
**GB**

(73) Proprietor: **UNILEVER N.V.
Weena 455
NL-3013 AL Rotterdam(NL)**

(84) Designated Contracting States:
**BE CH DE ES FR GR IT LI NL SE AT**

(72) Inventor: **Burger, Allan Robert
259 Passaic Avenue
Passaic, NJ 07055(US)**
Inventor: **Figdore, Phillip Eugene
379 Mountain Avenue
Franklin Lakes, NJ 07417(US)**
Inventor: **Lin, Samuel O.S.
76 Brown Circle
Paramus, NJ 07652(US)**
Inventor: **Massaro, Michael
270 Orchard Terrace
Bogota, NJ 07603(US)**

(74) Representative: **Ford, Michael Frederick et al
MEWBURN ELLIS 2 Cursitor Street
London EC4A 1BO (GB)**

**Description**

The invention relates to an antiperspirant in the form of a gelled stick, which is transparent.

Solid antiperspirant formulations generally fall within two broad categories - suspensoid sticks and gelled alcoholic sticks. Suspensoid sticks usually consist of acidic antiperspirant actives suspended in a matrix formed by a wax in an emollient. Illustrative of this category are the formulations presented in US patent 4,431,837 (Geria). Example 7 of this patent describes an antiperspirant stick suspending an aluminium/zirconium chlorohydroxide complex in a wax matrix of stearyl alcohol and castorwax. Volatile silicone and $C_8$-$C_{18}$ aliphatic hydrocarbon ethoxylated alcohol benzoates are included as emollients and vehicles for the composition. See also US patent 4,126,679 (Davy et al). Unfortunately, these sticks cannot be formulated into an attractive transparent appearance. The wax matrix and insolubility of the antiperspirant active render the stick opaque.

Two main formulating methods are known for gelled alcoholic sticks. One method is to utilize an alkaline aluminium chlorhydrate-lactate complex as the active and combine this with sodium stearate as a gelling agent. For instance, US patent 3,259,545 (Teller) reports a stick containing an aluminium antiperspirant active, sodium lactate and sodium stearate. US patent 3,472,940 (Osipow et al) obtains stable gelled alcoholic compositions through the use of sodium stearyl-2-lactylate. A second method for obtaining gelled alcoholic sticks involves use of an alcohol soluble aluminium chlorhydrate complex (e.g. an aluminium chlorhydrate-propylene glycol adduct) and dibenzaldehyde monosorbitol acetal as gelling agent. Illustrative is US patent 4,518,582 (Schamper et al) wherein solid transparent gelled sticks of the foregoing type are disclosed.

GB 2064363 (General Electric company) describes a silicone-water emulsion suitable as a vehicle for the application of epidermal enhancing agents, comprising 100 parts by weight of a specified category of cydic polysiloxanes, 0.7 to 666 parts by weight of an emulsifier, and 5 to 960 parts by weight of water.

Although gelled alcoholic sticks in transparent form can be achieved, these formulations suffer certain disadvantages. The alkaline aluminium active-lactate complex with sodium stearate is not particularly, effective because alkaline antiperspirant actives intrinsically have low efficacy. Alcohol soluble aluminium active complexes as found in Schamper et al provide unstable sticks because of the instability of acetal gelling agents in the presence of acidic aluminium actives. Many of the foregoing compositions form sticks that are not completely hardenable, thereby resulting in a tacky feel.

Accordingly, it is an object of the present invention to obtain an antiperspirant composition in stick form that is transparent and also has good antiperspirant properties.

Another object of the present invention (in preferred forms) is to obtain a transparent stick of acceptable hardness to avoid a tacky feel when applied to skin.

The present invention provides an antiperspirant composition in the form of a stick which is transparent, and comprises by weight:

(i) from 5 to 25% of an acidic aluminium salt with antiperspirant activity;

ii) from 10 to 40% of a nonionic surfactant;

iii) from 5 to 50% of a liquid oil immiscible with water; and

iv) from 20 to 50% water.

and wherein the quantities are selected so that the stick is transparent.

Aluminium chlorhydrate is most desirable as the antiperspirant salt. The nonionic surfactant is desirably, a $C_{11}$-$C_{18}$ alcohol ethoxylate. The liquid oil component may preferably be selected from emollient oils, volatile silicones and, desirably, mixtures of these materials.

We have discovered that acidic aluminium antiperspirant salts may be structured into a stick form which is transparent by use of nonionic surfactants. The basic composition comprises an oil and an aqueous phase. The aqueous phase contains the active, aluminium salt. Nonionic surfactant induces gelation to harden the composition. Transparency can be achieved by matching the refractive indices of the two phases.

When deciding upon a formulation, adjustment of refractive index is best performed by adjusting the refractive index of the oil phase.

Preferably volatile silicones are utilized in the oil phase to detackify the stick. Normally there is a significant difference in the refractive index of silicone and water phases. We have found that an emollient oil may be added to the silicone to adjust the refractive index of the oil phase and render it compatible with that of the aqueous phase.

The term "transparent" as used in this specification is intended to connote its usual dictionary definition. Thus, a transparent antiperspirant stick, like glass, allows ready viewing of objects behind it. By contrast, a translucent antiperspirant stick, although allowing light to pass through, causes the light to be so scattered that it will be impossible to see clearly objects behind the translucent stick.

Within the context of this invention, an antiperspirant stick is deemed to be transparent if the maximum transmittance of light of any wavelength in the range 400 to 800 nm through a sample 1cm thick is greater than 35%, but preferably at least 50%. A bar is deemed translucent if the maximum transmittance of such light through the sample is between 2% and less than 35%. Finally, a bar is deemed opaque if the maximum transmittance of such light is less than 2%. This transmittance can easily be measured by placing a stick sample of the required thickness into the light beam path of a spectrophotometer whose working range includes the visible spectrum such as a Bausch & Lomb Spectronic 88 Spectrophotometer.

Antiperspirant actives suitable for the present invention include astringent acidic aluminium compounds, especially aluminium chlorhydroxides. Among the effective actives are aluminium chlorhydrate, activated aluminium chlorhydrate (such as Reach 201, sold by the Reheis Company USA), as well as lower aluminium/chlorine ratio actives (e.g. dichlorohydrates and sesquichlorohydrates). The aluminium active(s) will generally be present in an amount from about 5 to 25% by weight of the composition, preferably from about 10 to 20%. Beyond 20% active, the composition becomes more tacky and the stick softer. Unexpectedly, zirconium containing actives cause the composition to lose transparency. Thus, materials such as those sold under the Trade Mark Rezal 36GP, Rezal 36 and Rezal 67P were found unsuitable. These zirconium-containing actives appear to be strong salting out electrolytes for the surfactant.

Surfactants of the present invention must be capable of forming clear or translucent ordered liquid crystalline phases in the presence of water. Particularly desirable are surfactants forming viscous isotropic (cubic) and middle (hexagonal) phases. There must be no interaction between the surfactant and the antiperspirant actives in a manner leading to precipitation. Thus anionic and amphoteric surfactants are unsuitable for the present compositions because of their incompatibility with the antiperspirant actives. Anionics and amphoterics, such as betaines, interact with the positively charged aluminium salts and thereby form a precipitating complex. By contrast, nonionic surfactants have been found not to adversely interact with the actives. These materials are also preferred because of their less irritating nature to the skin. It will be preferable to choose a nonionic surfactant which gives a rigid stick: this can readily be determined by experiment.

Nonionic surfactants particularly suitable for the present invention are alkoxylated derivatives of compounds containing $C_{11}$-$C_{22}$ fatty alkyl hydrophobic groups. Two categories of these type of materials are particularly effective. These are:

a) polyoxyethylene and/or polyoxypropylene condensates of aliphatic carboxylic acids, whether linear-or branched-chain and unsaturated or saturated, containing from about 11 to about 22 carbon atoms in the aliphatic chain and incorporating from about 7 to about 40 (notably from 7 or 10 to 20) ethylene oxide or propylene oxide units. Suitable carboxylic acids include "coconut" fatty acid which contains an average of about 12 carbon atoms, "tallow" fatty acid which contains an average of about 18 carbon atoms, palmitic acid, myristic acid, stearic acid and lauric acid.

b) polyoxyethylene and/or polyoxypropylene condensates of aliphatic alcohols, whether linear- or branched-chain and unsaturated or saturated, containing from about 11 to 22 carbon atoms and incorporating from about 7 to 40 (preferably 7 or 10 to 20) ethylene oxide and/or propylene oxide units. Suitable alcohols include the "coconut" fatty alcohol, "tallow" fatty alcohol, lauryl alcohol, myristic alcohol, and oleyl alcohol.

Most effective within the context of this invention are the $C_{11}$-$C_{18}$ fatty alcohols ethoxylated with from about 10 to about 20 moles ethylene oxide. Especially effective, and studied in detail, is Neodol 45-13 which is available from Shell and is a $C_{14}$-$C_{15}$ fatty alcohol ethoxylated with an average of 13 moles of ethylene oxide. Further preferred nonionic surfactants are $C_{18}$ fatty alcohol alkoxylated with average 20 moles ethylene oxide, which is available from ICI under the Trade Mark Brij 99, and $C_{16}$ fatty alcohol alkoxylated with average 16 moles ethylene oxide.

Amounts of the nonionic surfactant required for the compositions of this invention range from about 10 to 40%, preferably from about 25 to 35% by weight.

The compositions of the present invention must contain a liquid oil immiscible with water. Total liquid oil present will range from about 5 to 50% by weight, preferably from about 10 to 40% by weight. The liquid oil component may itself be composed of emollient oils, volatile silicones, and preferably mixtures thereof. Emollient oils are defined as liquids at room temperature being immiscible with water (and preferably miscible with volatile silicones). Among the emollients oils may be included linear and branched chain fatty acid esters, diesters of dicarboxylic acids, and liquid hydrocarbons. Examples of fatty acid esters include the isopropyl esters of myristic, palmitic and stearic acids. Branched chain fatty acid esters are illustrated by 2-butylhexylpalmitate and 2-ethylhexyloxystearate. Di-n-butyl phthalate and diisopropyladipate are exemplative of dicarboxylic acid diesters. Mineral oils and paraffins, such as are available from Exxon under the Trade Mark Isopar, are illustrative of suitable liquid hydrocarbons. Most preferred among the emollient oils is 2-ethylhexyloxystearate (the 2-ethylhexyl ester of a hydroxystearic acid) which is available as Wickenol 171 from the Wickhen Corporation. The amount of emollient oil present will usually be from about 2 to 30%, preferably from about 5 to 15% by weight of the composition.

Volatile silicones are present mainly to assist in detackifying the stick. These materials also provide a dry, non-oily lubricant effect when stick contacts skin. Volatile silicones are relatively low molecular weight cyclic siloxane oligomers. The most readily available species of these siloxanes are hexamethylcyclotrisiloxane (boiling point 134°C), octamethylcyclotrisiloxane (boiling point 175.8°C) and decamethylcyclopentasiloxane (boiling point 210°C), more commonly known as trimer ($D_3$), tetramer ($D_4$) and pentamer ($D_5$), respectively. The $D_4$ and $D_5$ materials are also known, under the terminology of the Cosmetics, Toiletry and Fragrance Association, Inc (CTFA) as "Cyclomethicone". Commercially the $D_5$ cyclomethicone is available from Union Carbide Corporation as VS7158 and as DC 344 from Dow Corning Corporation. The amounts of volatile silicone in the present compositions will usually range from about 5 to 30%, preferably from about 10 to 20% by weight.

Although volatile silicone can be utilized as the only component of the liquid oil phase, often there is a problem with matching the refractive indices of volatile silicone with the aqueous phase and other components of the composition. Therefore, it has been found useful to use a mixture of the volatile silicone and an appropriate emollient oil for adjustment of refractive index. When combinations of a volatile silicone and emollient oil are utilized, the ratios of these materials will normally range from about 10:1 to 1:10, respectively. Preferably, the respective ratio will range from about 4:1 to 1:4, optimally about 2:1 to 1:1.

For a given surfactant concentration, cosmetic properties of the stick, such as its hardness and tackiness, are dependent on both the nature and amount of the oil phase. In general, as the oil level increases, the stick becomes less tacky. However, hardness will increase initially but then decrease as the oil level is further increased.

Water is an important component of the composition. It must be present from about 20 to 50% by weight of the composition, preferably from about 25 to about 35% by weight. Amounts of water substantially lower than 20% result in sticks that are quite poor in hardness.

Other optional ingredients may be incorporated into the compositions of this invention. These ingredients include perfumes, preservatives, colorants and antimicrobial deodorizing agents (e.g. triclosan). These materials will usually be present in amounts less than 5% of each and usually less than 1%.

Antiperspirant sticks of the present invention may be prepared by any of four methods. First is a "normal addition method which involves adding the oil phase slowly to a solution of the surfactant in the aqueous antiperspirant active. Second is "reverse addition" wherein aqueous antiperspirant active is added to a mixture of surfactant and oil phase. Thirdly, it is possible to add surfactant to an emulsion of the aqueous phase containing antiperspirant active with the oil phase. Finally, it is possible to add such an emulsion of oil phase and aqueous phase containing active antiperspirant to the surfactant. Although all four methods can be used, the "reverse addition" procedure is preferred because of its simplicity and because it gives less foaming relative to, for instance, the "normal addition" method.

The following examples will more fully illustrate the embodiments of this invention. All parts, percentages and proportions referred to herein and in the appended claims are by weight unless otherwise indicated.

In these Examples "Neodol 45-13" denotes $C_{14}$-$C_{15}$ fatty alcohol ethoxylated with average 13 moles of ethylene oxide. "VS 7158" denotes decamethylcyclopentasiloxane, "Wickenol 171" denotes 2-ethylhexyloxystearate, "Isosteareth 20" denotes isostearyl alcohol ethoxylated with average 20 moles ethylene oxide and "Brij 78" denotes stearyl alcohol ethoxylated with average 20 moles ethylene oxide.

EXAMPLE 1

Illustrative of a typical preparation is that described as follows: Neodol 45-13, in the amount of 33 grams, was added to a thermostatted beaker maintained at 65-75°C. A mixture was prepared of 17 grams water with 30 grams of a 50% aqueous aluminium chlorohydrate solution. The combined water and aluminium chlorohydrate was then slowly added to the Neodol 45-13 with constant gentle agitation using a magnetic stirrer. Slow stirring was continued until most of the bubbles had dissipated. Refractive index of the solution was then measured.

Thereafter, a mixture was prepared of 12.8 grams VS 7158 (volatile silicone) and 7.2 grams Wickenol 171 (2-ethylhexyloxystearate). Refractive index of the oil phase was now measured. In those instances where the oil and water phases were not within approximately 0.001 units refractive index from one another, the ratio of VS 7158 : Wickenol 171 was readjusted by an increase in the appropriate oil to attain the equivalent refractive index. Next, the oil phase was slowly added with stirring to the surfactant/water/active phase. When most of the bubbles had dissipated, the composition was poured into moulds to cool.

EXAMPLES 2-6

A series of further compositions were prepared by the method outlined in Example 1. Details are given in Table 1 below. These compositions were used to evaluate the effect of volatile silicone and emollient oils.

## TABLE I

## Effect of Volatile Silicone and Emollient Oil

|  | Example (Parts by Weight) | | | | |
|---|---|---|---|---|---|
| Ingredient | 2 | 3 | 4 | 5 | 6 |
| Neodol 45-13 | 33 | 33 | 33 | 32 | 32 |
| Water | 32 | 32 | 31.96 | 33 | 33 |
| Aluminium Chlorohydrate | 15 | 15 | 15 | 10 | 10 |
| VS 7158 | 12.8 | }19* | 12.8 | 16 | 19 |
| Wickenol 171 | 7.2 | | 7.2 | 9 | -- |
| Perfume | -- | 1 | -- | -- | -- |
| FD&C Blue #1 (blue colour) | -- | -- | .04 | -- | -- |
| $C_{12}$-$C_{15}$ alkyl benzoate | -- | -- | -- | -- | 6 |

*Total VS 7158 + Wickenol 171 = 19%; exact ratio of VS 7158 to Wickenol 171 will depend on the refractive index of the perfume.

Examples 2 and 3, respectively, illustrate that the stick may be perfumed or dyed. Example 5 has less antiperspirant active and more oil than Example 2. This was found to result in a less tacky product. Example 6 is similar to Example 5 but illustrates the use of a different oil to adjust the oil phase refractive index. All Examples provided transparent compositions.

EXAMPLES 7-11

In Example 7 a composition identical to that of Example 1 was prepared but using the "reverse addition" method. The same method was used for Examples 8 to 11. All components of the oil phase were mixed and this was added slowly with stirring to the surfactant. Next, the antiperspirant active/water solution was slowly added to the surfactant/oil phase. The resultant emulsion was allowed to stand until the bubbles had dissipated. Compositions made thereby were then poured into molds. "Reverse addition" resulted in less foam and bubble problems than occurred in Example 1. The formulations prepared according to this method are outlined in Table II.

5

## TABLE II

### Example (Parts by Weight)

| Ingredient | 7 | 8 | 9 | 10 | 11 |
|---|---|---|---|---|---|
| Neodol 45-13 | 33 | 30 | 32 | 16.5 | -- |
| Water | 32 | 32 | 32 | 32 | 33 |
| Aluminium Chlorohydrate | 15 | 20 | 15 | 15 | 15 |
| VS 7158 | 12.8 | 10.8 | 20 | }20* | }20* |
| Wickenol 171 | 7.2 | 7.2 | -- | | |
| Isosteareth 20 | -- | -- | -- | -- | 32 |
| Brij 78 | -- | -- | -- | 16.5 | -- |

*Relative proportions of VS 7158 and Wickenol 171 adjusted to achieve transparency.

Example 8, relative to Example 7, contains an increased amount of antiperspirant active and less oil; the stick derived from Example 8 was more tacky than that of Example 7. The product of Example 9 was not transparent due to the mis-match of refractive indices of the phases. Examples 10 and 11 illustrate the use of mixed surfactants and a surfactant other than Neodol 45-13. Examples 7, 8, 10 and 11 were transparent.

EXAMPLES 12-26

Formulation effects upon stick hardness are illustrated in the following examples. In all cases, the oil phase was a mixture of VS 7158:Wickenol 171 (64:36).

## TABLE III

### Formulation Effects Upon Stick Hardness

| Example | % Neodol 45-13 | % Aluminium Chlorohydrate | % Water | % Oil | Relative Hardness |
|---------|----------------|---------------------------|---------|-------|-------------------|
| 12 | 22 | 15 | 22 | 41 | 0.30 |
| 13 | 24 | 15 | 24 | 37 | 0.39 |
| 14 | 28 | 15 | 47 | 10 | 0.48 |
| 15 | 28 | 15 | 37 | 20 | 0.63 |
| 16 | 28 | 15 | 27 | 30 | 0.44 |
| 17 | 28 | 15 | 22 | 35 | 0.30 |
| 18 | 31 | 15 | 34 | 20 | 0.89 |
| 19 | 33 | 15 | 47 | 5 | 0.41 |
| 20 | 33 | 15 | 42 | 10 | 0.89 |
| 21 | 33 | 15 | 37 | 15 | 1.00 |
| 22 | 33 | 15 | 32 | 20 | 1.00 |
| 23 | 33 | 15 | 25 | 27 | 0.63 |
| 24 | 33 | 15 | 14 | 38 | 0.04 |
| 25 | 35 | 15 | 40 | 10 | 1.33 |
| 26 | 35 | 15 | 30 | 20 | 0.89 |

Stick hardness was measured using an Instron Model 1122 Universal Testing Instrument, employing an 8.9mm penetrometer driven at 2mm/minute.

Examples 14-17 and 19-24 illustrate the effect of the amount of oil upon hardness when the amount of surfactant is 28% and 33% respectively. At both surfactant concentrations maximum hardness occurs when the amount of oil is around 20%. Other Examples in Table III investigate hardness values at surfactant concentrations lower and higher than 28-33%. Generally, hardness increases in direct proportion to the surfactant concentration. For instance, compare Examples 14, 20 and 25 where the oil concentration is constant at 10% and surfactant concentration is 28, 33 and 35% respectively. Relative hardness increases from 0.48 up to 1.33 as the surfactant is increased.

Examples 25 and 26 show that optimum hardness can occur at different concentrations of oil, depending on the surfactant concentration. For instance, the composition of Example 25 (10% oil) is harder than that of Example 26 (20% oil) at a 35% surfactant concentration. By contrast, at a 33% surfactant concentration, the 10% oil formulation of Example 20 is softer than the 20% oil formulation of Example 21.

### EXAMPLES 27-40

The following Examples illustrate the use of different types of nonionic surfactants and their effect upon relative hardness. All of the formulations contained 33% surfactant, 15% aluminium chlorohydrate, 32% water and 20% VS 7158/Wickenol 171 as the oil phase. Table IV outlines these formulations in which all surfactants are fatty alcohol ethoxylates:

### TABLE IV

### Effect of Various Surfactants on Stick Hardness

| Example | Surfactant Trade Mark | CTFA Name | No. of C Atoms in Fatty Alcohol | No. of EO Groups | Relative Hardness |
|---|---|---|---|---|---|
| 27 | Procol LA20 | Laureth 20 | 12 | 20 | 0.55 |
| 28 | Siponic TD 990 | Trideceth 9 | 13 | 9 | 0.09 |
| 29 | Macol TD 10 | Trideceth 10 | 13 | 10 | 0.19 |
| 30 | Renex 30 | Trideceth 12 | 13 | 12 | 0.27 |
| 31 | Neodol 45-13 | Pareth 45-13 | 14-15 | 13 | 1.00 |
| 32 | Tergitol 15-S-20 | Pareth 15-20 | 11-15 | 20 | 0.37 |
| 33 | Brij 56 | Ceteth 10 | 16 | 10 | 0.41 |
| 34 | Procol CA16 | Ceteth 16 | 16 | 16 | 0.85 |
| 35 | Procetyl AWS | PPG5-Ceteth 20 | 16 | 20* | 0.44 |
| 36 | Procol CS-20 | Cetereth 20 | 16/18 | 20 | 0.52 |
| 37 | Brij 76 | Steareth 10 | 18 | 10 | 0.06 |
| 38 | Arosurf 66E10 | Isosteareth 10 | 18 (iso) | 10 | 0.55 |
| 39 | Brij 97 | Oleth 10 | 18 (oleyl) | 10 | 0.48 |
| 40 | Brij 99 | Oleth 20 | 18 (oleyl) | 20 | 0.76 |

*Co-alkoxylated with 5 propylene oxide groups.

From Table IV, it appears that most $C_{11}$-$C_{18}$ alcohol ethoxylates impart some degree of hardness to the stick. It is, however, noted that Neodol 45-13, Procol CA16 and Brij 99 are particularly effective.

EXAMPLES 41-59

In these Examples there are illustrated surfactants which do not result in a hard material: liquid phase or soft gels were obtained with these surfactants. Again, all of the formulations contain 33% surfactant, 15% aluminium chlorhydrate, 32% water and 20% VS 7158/Wickenol 171 as the oil phase. Table V lists the ineffective surfactants.

Some of the surfactants are fatty alcohol ethoxylates and for these Table V quotes the number of carbon atoms in the fatty alcohol group and the average number of ethylene oxide residues per molecule.

8

## TABLE V

### Surfactants Not Providing Hard Sticks

| Example | Surfactant Trade Mark | CTFA Name | Nature of/ C atoms in Hydrophobe Group | No of EO Groups |
|---------|----------------------|-----------|----------------------------------------|-----------------|
| 41 | Sandoz SX-408 | Pending* | 10 (iso) | 4 |
| 42 | Sandoz SX-412 | Pending* | 10 (iso) | 6 |
| 43 | Sandoz SX-418 | Pending* | 10 (iso) | 9 |
| 44 | Sandoz SX-424 | Pending* | 10 (iso) | 12 |
| 45 | Brij 721 | Steareth 21 | 18 | 21 |
| 46 | Triton X-100 | Octoxynol 9 | alkylphenol | 9 |
| 47 | Tergitol 24-L-25N | Pareth-25 | 12-14 | 25 |
| 48 | Tergitol 24-L-35N | Pareth-35 | 12-14 | 35 |
| 49 | Tergitol 24-L-50N | Pareth-50 | 12-14 | 50 |

| 50 | Polychol 20 | Laneth 20 | lanolin alcohol | 20 |
| 51 | Emerest 2712 | PEG-8-distearate | stearic acid | 8 |
| 52 | Emerest 2715 | PEG-40-distearate | stearic acid | 40 |
| 53 | Mapeg CO25H | PEG-25 Hydrogenated | hydrogenated castor oil | 25 |
| 54 | Barlox 14 | | myristamine oxide | |
| 55 | Schercomid CCD | | coconut diethanolamide | |
| 56 | Carsamide CMEA | | lauric diethanolamide | |
| 57 | Lonzaine CZ | | cocoamidosulfobetaine | |
| 58 | Pluronic F127 | Poloxamer 407 | polyoxypropylene polyoxyethylene block copolymer | |
| 59 | Pluronic L72 | Poloxamer 212 | polyoxypropylene polyoxyethylene block copolymer | |

*These fatty alcohol ethoxylates each contain 2 propylene groups in addition to the ethylene oxide groups on the $C_{10}$ alkyl chain.

EXAMPLES 60-73

The following Examples, detailed in Table VI below, illustrate the effect of various liquid oils on the properties of the stick as described in Example 1. All formulations contain 33% Neodol 45-13, 15% aluminium chlorohydrate, 32% water and 20% of the oil phase. These formulations were not adjusted to transparency but merely formulated to achieve adequate hardness.

## TABLE VI

### Effect of Various Liquid Oils

| Example | Oil | Relative Hardness |
|---|---|---|
| 60 | VS 7158 | 0.52 |
| 61 | 2-Ethylhexyloxystearate | 0.70 |
| 62 | 64:36, VS 7158:2-ethyl-hexyloxystearate | 1.00 |
| 63 | Isopropyl Palmitate | 1.04 |
| 64 | Isopropyl Myristate | 0.9 |
| 65 | Isopropyl Stearate | 1.1 |
| 66 | Butyl Myristate | 0.85 |
| 67 | 2-Ethyhexyl Palmitate | 1.00 |
| 68 | 2-Ethylhexyl Stearate | 1.2 |
| 69 | Isopar C ($C_7$-$C_8$ isoparaffin, bp 99°C) | 0.96 |
| 70 | Isopar M ($C_{13}$-$C_{14}$ isoparaffin, bp 223°C) | 1.07 |
| 71 | $C_{12}$-$C_{15}$ Benzoate (Finsolv TN) | 0.15 |
| 72 | Myristyl Octanoate | 0.85 |
| 73 | Di-2-ethylhexyl Succinate | 0.63 |

EXAMPLE 74

In this Example, the effect of zirconium containing actives was evaluated. A composition containing 14 grams aluminium chlorohydrate, 33 grams Neodol 45-13 and 32 grams water was formulated with 1 gram of each of three zirconium/aluminium antiperspirant actives, with aluminium chlorohydrate used in a control. The results are recorded in Table VII. It is evident that the presence of zirconium renders the composition non-transparent.

## TABLE VII

### Effect of Zirconium/Aluminium Active on Transparency

| Antiperspirant Active | Composition (weight) | | | |
|---|---|---|---|---|
| | A | B | C | D |
| Aluminium Chlorohydrate | 15 | 14 | 14 | 14 |
| Rezal 36GP* | -- | 1 | -- | -- |
| Rezal 36* | -- | -- | 1 | -- |
| Rezal 67GP* | -- | -- | -- | 1 |
| Appearance: | Clear | Cloudy | Cloudy | Cloudy |

*Contains Zirconium as part of the active system.

EXAMPLE 75

Herein is illustrated the difference in clarity between different stick formulations. Clarity was determined by measuring the transmittance of a 1.0cm thick section of a stick formulation by use of a Bausch & Lomb Spectronic 88 Spectrophotometer. Table VIII sets forth a pair of formulations, one transparent and the other translucent. These formulations were then measured for transmittance and compared with a commercial opaque stick. Results are given in Table IX.

## TABLE VIII

### Formulations Measured for Transmittance

| Ingredient | Transparent Formula Weight % | Translucent Formula Weight % |
|---|---|---|
| Neodol 45-13 | 33.0 | 33.0 |
| VS 7158 | 12.8 | 10.0 |
| Wickenol 171 | 7.2 | 10.0 |
| Aluminium Chlorohydrate | 15.0 | 15.0 |
| $H_2O$ | 32.0 | 32.0 |

## TABLE IX

### Transmittance Values of Antiperspirant Stick Formulations

| Wavelength (nm) | Transparent Formulation (Transmittance%) | Translucent Formulation (Transmittance%) | Opaque Commercial Stick (Transmittance%) |
|---|---|---|---|
| 400 | 48.0 | 2.5 | 0 |
| 450 | 50.0 | 3.5 | 0 |
| 500 | 52.0 | 5.0 | 0 |
| 550 | 54.0 | 6.0 | 0 |
| 600 | 57.5 | 7.5 | 0 |
| 650 | 61.0 | 9.5 | 0 |
| 700 | 65.5 | 11.0 | 0 |
| 750 | 69.0 | 12.5 | 0 |
| 800 | 73.0 | 14.0 | 0 |

**Claims**

1. An antiperspirant composition in the form of a stick which comprises by weight:
   i) from 5 to 25% of an acidic aluminium salt with antiperspirant activity;
   ii) from 10 to 40% of a nonionic surfactant;
   iii) from 5 to 50% of a liquid oil immiscible with water; and
   iv) from 20 to 50% water;
   and wherein the quantities are selected so that the stick is transparent.

EP 0 291 334 B1

**2.** An antiperspirant composition according to claim 1 wherein the liquid oil is one or more emollient oils, volatile silicones or mixtures thereof.

**3.** An antiperspirant composition according to claim 2 wherein the emollient oil(s) are selected from linear and branched chain fatty acid esters, diesters of dicarboxylic acids, liquid hydrocarbons and mixtures thereof.

**4.** An antiperspirant composition according to claim 2 wherein the emollient oil is 2-ethylhexyloxystearate.

**Patentansprüche**

**1.** Schweißhemmende Zusammensetzung in Form eines Stiftes, die bezogen auf Gewicht umfaßt:
    i. 5 bis 25 % eines sauren Aluminiumsalzes mit schweißhemmender Aktivität;
    ii. 10 bis 40 % eines nichtionischen Tensids;
    iii. 5 bis 50 % eines mit Wasser nicht mischbaren flüssigen Öls und
    iv. 20 bis 50 % Wasser,
    wobei die Mengen jeweils so ausgewählt werden, daß der Stift transparent ist.

**2.** Schweißhemmende Zusammensetzung nach Anspruch 1, worin das flüssige Öl ein oder mehrere weichmachende Öle, flüchtige Silikone oder Mischungen davon umfaßt.

**3.** Schweißhemmende Zusammensetzung nach Anspruch 2, worin das/die weichmachende(n) Öl(e) ausgewählt ist/sind aus linearen und verzweigten Fettsäureestern, Diestern von Dicarbonsäuren, flüssigen Kohlenwasserstoffen und Mischungen davon.

**4.** Schweißhemmende Zusammensetzung nach Anspruch 2, worin das weichmachende Öl 2-Ethylhexyloxystearat ist.

**5.** Schweißhemmende Zusammensetzung nach einem der Ansprüche 2 bis 4, worin das/die weichmachende(n) Öl(e) in einer Menge von 2 bis 30 Gew.-% vorhanden ist/sind.

**6.** Schweißhemmende Zusammensetzung nach einem der Ansprüche 2 bis 5, worin das/die flüchtige(n) silikon(e) in einer Menge von 5 bis 30 Gew.-% vorhanden ist/sind.

**7.** Schweißhemmende Zusammensetzung nach einem der Ansprüche 2 bis 6 umfassend eine Mischung eines flüchtigen silikons oder flüchtiger Silikone mit einem weichmachenden Öl oder weichmachenden Ölen in einem Verhältnis von 10:1 bis 1:10.

**8.** Schweißhemmende Zusammensetzung nach einem der vorhergehenden Ansprüche, worin das nichtionische Tensid ein alkoxyliertes Derivat einer Verbindung oder von Verbindungen ist, die $C_{11}$-$C_{22}$-Fettalkylgruppen enthalten.

**9.** Schweißhemmende Zusammensetzung nach Anspruch 8, worin das nichtionische Tensid ein $C_{11}$-$C_{18}$-Fettalkohol ist, der mit 10 bis 20 Mol Ethylenoxid alkoxyliert ist.

**10.** Schweißhemmende Zusammensetzung nach einem der vorhergehenden Ansprüche, worin das nichtionische Tensid in einer Menge von 25 bis 35 Gew.-% vorhanden ist.

**Revendications**

**1.** Une composition déodorante sous la forme d'un bâtonnet comprenant:
    (i) de 5 à 25 % en masse d'un sel acide d'aluminium ayant une activité déodorante;
    (ii) de 10 à 40 % en masse d'un agent tensioactif non ionique;
    (iii) de 5 à 50 % en masse d'une huile liquide non miscible avec l'eau ; et
    (iv) de 20 à 50% en masse d'eau,
    et dans laquelle les quantités sont choisies de telle sorte que le bâtonnet soit transparent.

14

**2.** Une composition déodorante selon la revendication 1, dans laquelle l'huile liquide est une ou plusieurs huiles émollientes, silicones volatiles ou des mélanges de celles-ci.

**3.** Une composition déodorante selon la revendication 2, dans laquelle les huiles émollientes sont sélectionnées à partir des esters et des diesters gras à chaîne droite ou ramifiée d'acides dicarboxyliques, des hydrocarbures liquides et des mélanges de ceux-ci.

**4.** Une composition déodorante selon la revendication 2, dans laquelle l'huile émolliente est du 2-éthylhexylstéarate.

**5.** Une composition déodorante selon l'une des revendications 2 à 4, dans laquelle l'huile (les huiles) émolliente(s) est (sont) présente(s) dans une quantité allant de 2 à 30 % en masse.

**6.** Une composition déodorante selon l'une des revendications 2 à 5, dans laquelle la (les) silicone(s) volatile(s) est (sont) présente(s) dans une quantité allant de 5 à 30 % en masse.

**7.** Une composition déodorante selon l'une des revendication 2 à 6 comprenant un mélange de silicone(s) volatile(s) et d'huile(s) émolliente(s) dans un rapport allant de 10 pour 1 à 1 pour 10.

**8.** Une composition déodorante selon l'une des revendications précédentes, dans laquelle l'agent tensioactif non ionique est un dérivé alkoxylé d'un ou de plusieurs composés contenant des groupes alkyles gras en $C_{11}$-$C_{22}$.

**9.** Une composition déodorante selon la revendication 8, dans laquelle l'agent tensioactif non ionique est un alcool gras en $C_{11}$-$C_{18}$ alkoxylé avec de 10 à 20 moles d'oxyde d'éthylène.

**10.** Une composition déodorante selon l'une des revendications précédentes, dans laquelle l'agent tensioactif non ionique est présent dans une quantité allant de 25 à 35 % en masse.